**Europäisches Patentamt**

**European Patent Office**

**Office européen des brevets**

(19)

(11) Publication number : **0 471 466 A2**

(12) # EUROPEAN PATENT APPLICATION

(21) Application number : **91306897.9**

(22) Date of filing : **29.07.91**

(51) Int. Cl.⁵ : **C12N 15/53, // (C12N15/53, C12R1:66, 1:80, 1:745)**

(30) Priority : **03.08.90 US 562529**

(43) Date of publication of application :
**19.02.92 Bulletin 92/08**

(84) Designated Contracting States :
**AT BE CH DE DK ES FR GB GR IT LI NL SE**

(71) Applicant : **ELI LILLY AND COMPANY**
**Lilly Corporate Center**
**Indianapolis Indiana 46285 (US)**

(72) Inventor : **Gustafson, Gary David**
**8236 Tern Court**
**Indianapolis, Indiana 46256 (US)**
Inventor : **Waldron, Clive**
**6206 Green Leaves Road**
**Indianapolis, Indiana 46220 (US)**

(74) Representative : **Hudson, Christopher Mark et al**
**Erl Wood Manor**
**Windlesham Surrey GU20 6PH (GB)**

(54) **DNA sequences which impart resistance to the fungicide 8-chloro-4-(2-chloro-4-fluorophenoxy)quinoline.**

(57)   The present invention provides 8-chloro-4-(2-chloro-4-fluorophenoxy)quinoline (a fungicide) resistance genes for use as selectable markers in fungi. These resistance genes are mutants of the wild-type Aspergillus nidulans dihydroorotate dehydrogenase gene. The wild-type gene is also provided herein.

EP 0 471 466 A2

Jouve, 18, rue Saint-Denis, 75001 PARIS

The fungicide 8-chloro-4-(2-chloro-4-fluorophenoxy)quinoline (CCFQ) completely inhibits the fungal pathogens Botrytis cinerea and Penicillium digitalum at 50 μg/ml in greenhouse tests. The fungicide has an $IC_{50}$ (concentration that inhibits growth by 50%) of 0.15 μg/ml against Aspergillus nidulans. The fungi Cephalosporium and Neurospora are also sensitive to 8-chloro-4-(2-chloro-4-fluorophenoxy)quinoline.

There are few known resistance genes useful as selectable markers in the fungi. The present invention provides new DNA sequences which impart CCFQ resistance to fungi. The DNA sequences comprise mutations in the dihydroorotate dehydrogenase (DHOD) gene of Aspergillus nidulans. Also provided is the wild-type DHOD gene, which is useful for preparing other resistance genes.

The present invention provides DNA sequences that impart resistance to the fungicide 8-chloro-4-(2-chloro-4-fluorophenoxy)quinoline (CCFQ). These DNA sequences comprise mutations of the dihydroorotate dehydrogenase gene of Aspergillus nidulans. Also provided by the present invention is the wild-type DHOD gene, which is useful to create other mutant genes. The resistance genes are useful as selectable markers in fungi including, but not limited to, Aspergillus, Penicillium and Cephalosporium. Methods for constructing recombinant host cells resistant to CCFQ are also provided by the present invention, as are said recombinant host cells.

The following section provides a more detailed description of the present invention. For purposes of clarity and as an aid in the understanding of the invention the following items are defined below.

ApR - the ampicillin resistance-conferring gene.

CCFQ R - an 8-chloro-4-(2-chloro-4-fluorophenoxy)quinoline resistance gene.

cos - a DNA sequence required for packaging DNA into bacteriophage lambda particles.

Isolated DNA sequence - Any DNA sequence, however constructed or synthesized, which is locationally distinct from its location in genomic DNA. The definition includes the isolated DNA sequence in all its forms other than the natural state. For example, the DNA sequence may be inserted into a plasmid or phage vector or inserted into the genome of the organism from which it came or any other organism.

The following one-letter amino acid codes are used throughout the document.

| Symbol | Meaning |
|--------|---------|
| A | Alanine |
| C | Cysteine |
| D | Aspartic Acid |
| E | Glutamic Acid |
| F | Phenylalanine |
| G | Glycine |
| H | Histidine |
| I | Isoleucine |
| K | Lysine |
| L | Leucine |
| M | Methionine |
| N | Asparagine |
| P | Proline |
| Q | Glutamine |
| R | Arginine |
| S | Serine |
| T | Threonine |
| V | Valine |
| W | Tryptophan |
| Y | Tyrosine |

The restriction site and function maps presented in the accompanying drawings are approximate representations of the recombinant DNA vectors discussed herein. The restriction site information is not exhaustive; therefore, there may be more restriction sites of a given type on the vector than actually shown on the map.

Figure 1. A restriction site and function map of cosmid pKBY2.

Figure 2. A restriction site and function map of cosmid pGAR150.

Figure 3. A restriction map of the DHOD gene.

The present invention provides DNA compounds comprising isolated DNA sequences encoding 8-chloro-4-(2-chloro-4-fluorophenoxy)quinoline resistance genes. The isolated DNA sequences comprise mutations of the dihydroorotate dehydrogenase gene of Aspergillus nidulans. The DNA sequences are useful as selectable markers in fungi, especially Aspergillus Penicillium and Cephalosporium. Also provided by the present invention are methods for constructing a recombinant host cell resistant to 8-chloro-4-(2-chloro-4-fluorophenoxy)quinoline and the recombinant host cells so constructed. Finally, the dihydroorotate dehydrogenase gene of Aspergillus nidulans is also a part of the present invention.

The fungicide 8-chloro-4-(2-chloro-4-fluorophenoxy)quinoline (CCFQ) completely inhibits the growth of such fungal pathogens as Botrytis cinerea and Penicillium digitalum. CCFQ also inhibits the growth of Aspergillus, Neurospora and Cephalosporium. DNA sequences which impart resistance to CCFQ are useful as selectable markers in fungi. Such DNA sequences are mutants of the wild-type dihydroorotate dehydrogenase (DHOD) gene of Aspergillus nidulans. The wild-type DHOD gene is useful to make other resistance genes.

CCFQ is made by the procedures described in European Patent Publication No. 326330, published August 2, 1989. For use with Aspergillus, it is included in the media at 2.5 µg/ml.

Host cells comprising the wild-type DHOD gene are sensitive to CCFQ. The DNA sequence of this gene is listed below. All DNA sequences herein are depicted from left to right in the 5′ → 3′ orientation.

```
  1   ATGGCTACGA ATTCTTTCCG AAAACTCACT TTTTCAGGAG CCTCCCGTCT

 51   GGGTGGTTGT CGCCGTCTCC CACTAACCTG CAGACAACTT CGATTCGCCT

101   CCGACAGCGG AGCCGCAGCG GCAACTACAA AAGCAACGGC CGAATCAGCA

151   GCCGAGTCAG CTAGTATAAA CGTCAAAGAG GCACCCAAAA AGGCCGGACG

201   GGGCCTGCGG CGCACGGTCC TGGGAACGTC GTTGGCGCTG ACGCTGCTGG

251   TTGGATATGT CTACGGGACG GACACCCGGG CGAGTGTGCA TCGGTACGGT

301   GTTGTGCCGC TGATTAGAGC ATTGTATCCT GATGCGGAAG ATGCGCATCA

351   TATTGGTGTC GATACTTTAA AGATGCTGTA TAAGTATGGT CTGCATCCAA

401   GGGAACGGGG GGATCCGGAT GGAGATGGGG CGCTGGCGAC AGAG̊GTGGGT

451   GGTCTTTTAT TGTGATGGAA GTTAAGGCCC TAATACTTAT GGCGGATÅGG

501   TCTTTGGGTA TACACTGTCA AACCCAATTG GCATATCGGG CGGCCTGGAC

551   AAGCATGCTG AGATCCCTGA TCCGCTGTTC GCGATCGGTC CTGCCATTGT

601   CGAAGTCGGG GGTACGACAC CCTTACCACA GGATGGTAAC CCGCGTCCTC

651   GCGTATTCCG ACTTCCATCA CAGAGAGCGA TGATAAACCG GTACGGCCTC

701   AACTCCAAAG GCGCAGATCA CATGGCAGCT ATCTTGGAGC AACGAGTACG

751   CGATTTTGCC TACGCAAACG GATTTGGGGC ATACGATGCG GCTAAGCAGC
```

```
 801  GTGTATTGGA  CGGCGAAGCT  GGTGTGCCAC  CAGGTAGTCT  TCAGCCTGGT
 851  AAGCTTTTAG  CTGTCCAAGT  GGCAAAGAAC  AAGGCCACTC  CTGACGGCGA
 901  CATTGAAGCC  ATCAAGCGCG  ACTATGTGTA  TTGCGTGGAC  CGTGTGGCCA
 951  AATACGCTGA  TATTCTTGTT  GTGAATGTAT  CGAGCCCCAA  CACACCCGGT
1001  CTCCGTGACC  TTCAAGCCAC  TGCCCCGCTC  ACAGCTATCT  TGAGTGCTGT
1051  CGTTGGCGCG  GCAAAGAGCG  TGAACCGCAA  GACCAAACCA  TATGTTATGG
1101  TCAAGGTCAG  TCCGGATGAA  GACTCAGATG  AACAAGTCTC  TGGTATCTGC
1151  GACGCCGTCC  GAGCATCCGG  TGTCGACGGA  GTGATTGTCG  GAAACACAAC
1201  AAACCGTCGC  CCCGACCCTA  TACCCCAAGG  TTACACTCTT  CCGGCCAAGG
1251  AGCAGGCAAC  GTTGAAAGAA  ACCGGCGGGT  ATTCAGGTCC  ACAGCTGTTC
1301  GATCGCACAG  TGGCCCTTGT  GGCTCGGTAC  CGCTCCATGC  TGGATGCGGA
1351  GTCGGAAACG  GCCGGATCCG  CCAAGGATTC  AGCAGCGACC  ATAGCGCAAA
1401  CAGAGCCAGG  CTCGGAAAAC  GTTCCTCCTG  TGGAAGCGCC  AAGCGGACTG
1451  CCGCGCAAAG  TTATCTTCGC  TTCGGGTGGT  ATCACCAACG  GGAAGCAGGC
1501  TCACGCTGTT  TTAGACACAG  GGGCATCTGT  TGCCATGATG  TACACCGGTG
1551  TGGTCTATGG  TGGCGTCGGC  ACTGTCACTC  GAGTGAAGCA  AGAACTTCGA
1601  ACGGCGAAAA  AGGAG
```

The beginning and end of the single intron are marked with asterisks at the G at position 445 and the G at position 499 respectively.

The sequence of the gene with the intron removed is

```
  1  ATGGCTACGA  ATTCTTTCCG  AAAACTCACT  TTTTCAGGAG  CCTCCCGTCT
 51  GGGTGGTTGT  CGCCGTCTCC  CACTAACCTG  CAGACAACTT  CGATTCGCCT
101  CCGACAGCGG  AGCCGCAGCG  GCAACTACAA  AAGCAACGGC  CGAATCAGCA
151  GCCGAGTCAG  CTAGTATAAA  CGTCAAAGAG  GCACCCAAAA  AGGCCGGACG
201  GGGCCTGCGG  CGCACGGTCC  TGGGAACGTC  GTTGGCGCTG  ACGCTGCTGG
251  TTGGATATGT  CTACGGGACG  GACACCCGGG  CGAGTGTGCA  TCGGTACGGT
301  GTTGTGCCGC  TGATTAGAGC  ATTGTATCCT  GATGCGGAAG  ATGCGCATCA
```

4

```
 351   TATTGGTGTC GATACTTTAA AGATGCTGTA TAAGTATGGT CTGCATCCAA
 401   GGGAACGGGG GGATCCGGAT GGAGATGGGG CGCTGGCGAC AGAGGTCTTT
 451   GGGTATACAC TGTCAAACCC AATTGGCATA TCGGGCGGCC TGGACAAGCA
 501   TGCTGAGATC CCTGATCCGC TGTTCGCGAT CGGTCCTGCC ATTGTCGAAG
 551   TCGGGGGTAC GACACCCTTA CCACAGGATG GTAACCCGCG TCCTCGCGTA
 601   TTCCGACTTC CATCACAGAG AGCGATGATA AACCGGTACG GCCTCAACTC
 651   CAAAGGCGCA GATCACATGG CAGCTATCTT GGAGCAACGA GTACGCGATT
 701   TTGCCTACGC AAACGGATTT GGGGCATACG ATGCGGCTAA GCAGCGTGTA
 751   TTGGACGGCG AAGCTGGTGT GCCACCAGGT AGTCTTCAGC CTGGTAAGCT
 801   TTTAGCTGTC CAAGTGGCAA AGAACAAGGC CACTCCTGAC GGCGACATTG
 851   AAGCCATCAA GCGCGACTAT GTGTATTGCG TGGACCGTGT GGCCAAATAC
 901   GCTGATATTC TTGTTGTGAA TGTATCGAGC CCCAACACAC CCGGTCTCCG
 951   TGACCTTCAA GCCACTGCCC CGCTCACAGC TATCTTGAGT GCTGTCGTTG
1001   GCGCGGCAAA GAGCGTGAAC CGCAAGACCA AACCATATGT TATGGTCAAG
1051   GTCAGTCCGG ATGAAGACTC AGATGAACAA GTCTCTGGTA TCTGCGACGC
1101   CGTCCGAGCA TCCGGTGTCG ACGGAGTGAT TGTCGGAAAC ACAACAAACC
1151   GTCGCCCCGA CCCTATACCC CAAGGTTACA CTCTTCCGGC CAAGGAGCAG
1201   GCAACGTTGA AAGAAACCGG CGGGTATTCA GGTCCACAGC TGTTCGATCG
1251   GACAGTGGCC CTTGTGGCTC GGTACCGCTC CATGCTGGAT GCGGAGTCGG
1301   AAACGGCCGG ATCCGCCAAG GATTCAGCAG CGACCATAGC GCAAACAGAG
1351   CCAGGCTCGG AAAACGTTCC TCCTGTGGAA GCGCCAAGCG GACTGCCGCG
1401   CAAAGTTATC TTCGCTTCGG GTGGTATCAC CAACGGGAAG CAGGCTCACG
1451   GTGTTTTAGA CACAGGGGCA TCTGTTGCCA TGATGTACAC CGGTGTGGTC
1501   TATGGTGGCG TCGGCACTGT CACTCGAGTG AAGCAAGAAC TTCGAACGGC
1551   GAAAAAGGAG
```

The above DNA sequences encode the DHOD enzyme, which has the amino acid sequence:

```
  1   MATNSFRKLT FSGASRLGGC RRLPLTCRQL RFASDSGAAA ATTKATAESA
 51   AESASINVKE APKKAGRGLR RTVLGTSLAL TLLVGYVYGT DTRASVHRYG
101   VVPLIRALYP DAEDAHHIGV DTLKMLYKYG LHPRERGDPD GDGALATEVF
151   GYTLSNPIGI SGGLDKHAEI PDPLFAIGPA IVEVGGTTPL PQDGNPRPRV
201   FRLPSQRAMI NRYGLNSKGA DHMAAILEQR VRDFAYANGF GAYDAAKQRV
251   LDGEAGVPPG SLQPGKLLAV QVAKNKATPD GDIEAIKRDY VYCVDRVAKY
301   ADILVVNVSS PNTPGLRDLQ ATAPLTAILS AVVGAAKSVN RKTKPYVMVK
351   VSPDEDSDEQ VSGICDAVRA SGVDGVIVGN TTNRRPDPIP QGYTLPAKEQ
401   ATLKETGGYS GPQLFDRTVA LVARYRSMLD AESETAGSAK DSAATIAQTE
451   PGSENVPPVE APSGLPRKVI FASGGITNGK QAHAVLDTGA SVAMMYTGVV
501   YGGVGTVTRV KQELRTAKKE
```

Two mutations of the DHOD gene have been isolated which impart resistance to CCFQ. The preferred mutation changes the thymidyl residue at position 599 of the intron-less coding sequence and position 654 of the intron-containing sequence to an adenyl residue. The resultant amino acid change at position 200 is from valine to glutamic acid. The DNA sequence of the preferred resistance gene is:

```
   1  ATGGCTACGA ATTCTTTCCG AAAACTCACT TTTTCAGGAG CCTCCCGTCT
  51  GGGTGGTTGT CGCCGTCTCC CACTAACCTG CAGACAACTT CGATTCGCCT
 101  CCGACAGCGG AGCCGCAGCG GCAACTACAA AAGCAACGGC CGAATCAGCA
 151  GCCGAGTCAG CTAGTATAAA CGTCAAAGAG GCACCCAAAA AGGCCGGACG
 201  GGGCCTGCGG CGCACGGTCC TGGGAACGTC GTTGGCGCTG ACGCTGCTGG
 251  TTGGATATGT CTACGGGACG GACACCCGGG CGAGTGTGCA TCGGTACGGT
 301  GTTGTGCCGC TGATTAGAGC ATTGTATCCT GATGCGGAAG ATGCGCATCA
 351  TATTGGTGTC GATACTTTAA AGATGCTGTA TAAGTATGGT CTGCATCCAA
 401  GGGAACGGGG GGATCCGGAT GGAGATGGGG CGCTGGCGAC AGAG͙GTGGGT
 451  GGTCTTTTAT TGTGATGGAA GTTAAGGCCC TAATACTTAT GGCGGATA͙GG
 501  TCTTTGGGTA TACACTGTCA AACCCAATTG GCATATCGGG CGGCCTGGAC
 551  AAGCATGCTG AGATCCCTGA TCCGCTGTTC GCGATCGGTC CTGCCATTGT
 601  CGAAGTCGGG GGTACGACAC CCTTACCACA GGATGGTAAC CCGCGTCCTC
 651  GCGAATTCCG ACTTCCATCA CAGAGAGCGA TGATAAACCG GTACGGCCTC
 701  AACTCCAAAG GCGCAGATCA CATGGCAGCT ATCTTGGAGC AACGAGTACG
 751  CGATTTTGCC TACGCAAACG GATTTGGGGC ATACGATGCG GCTAAGCAGC
 801  GTGTATTGGA CGGCGAAGCT GGTGTGCCAC CAGGTAGTCT TCAGCCTGGT
 851  AAGCTTTTAG CTGTCCAAGT GGCAAAGAAC AAGGCCACTC CTGACGGCGA
 901  CATTGAAGCC ATCAAGCGCG ACTATGTGTA TTGCGTGGAC CGTGTGGCCA
 951  AATACGCTGA TATTCTTGTT GTGAATGTAT CGAGCCCCAA CACACCCGGT
1001  CTCCGTGACC TTCAAGCCAC TGCCCCGCTC ACAGCTATCT TGAGTGCTGT
1051  CGTTGGCGCG GCAAAGAGCG TGAACCGCAA GACCAAACCA TATGTTATGG
1101  TCAAGGTCAG TCCGGATGAA GACTCAGATG AACAAGTCTC TGGTATCTGC
1151  GACGCCGTCC GAGCATCCGG TGTCGACGGA GTGATTGTCG GAAACACAAC
1201  AAACCGTCGC CCCGACCCTA TACCCCAAGG TTACACTCTT CCGGCCAAGG
1251  AGCAGGCAAC GTTGAAAGAA ACCGGCGGGT ATTCAGGTCC ACAGCTGTTC
1301  GATCGCACAG TGGCCCTTGT GGCTCGGTAC CGCTCCATGC TGGATGCGGA
1351  GTCGGAAACG GCCGGATCCG CCAAGGATTC AGCAGCGACC ATAGCGCAAA
1401  CAGAGCCAGG CTCGGAAAAC GTTCCTCCTG TGGAAGCGCC AAGCGGACTG
1451  CCGCGCAAAG TTATCTTCGC TTCGGGTGGT ATCACCAACG GGAAGCAGGC
1501  TCACGCTGTT TTAGACACAG GGGCATCTGT TGCCATGATG TACACCGGTG
1551  TGGTCTATGG TGGCGTCGGC ACTGTCACTC GAGTGAAGCA AGAACTTCGA
1601  ACGGCGAAAA AGGAG
```

This DNA sequence is comprised by plasmid pGAR150, available from the NRRL, (Northern Regional Research Center (NRRL), Agricultural Research Service, U.S. Department of Agriculture, Peoria, Illinois, 61604) under accession number NRRL B-18692. Other DNA sequences of the invention may be derived from pGAR150 by well-known mutagenesis techniques, including site-specific and deletion mutagenesis. The coding sequence of the preferred resistance gene (with the intron removed) is:

```
   1  ATGGCTACGA ATTCTTTCCG AAAACTCACT TTTTCAGGAG CCTCCCGTCT
  51  GGGTGGTTGT CGCCGTCTCC CACTAACCTG CAGACAACTT CGATTCGCCT
 101  CCGACAGCGG AGCCGCAGCG GCAACTACAA AAGCAACGGC CGAATCAGCA
 151  GCCGAGTCAG CTAGTATAAA CGTCAAAGAG GCACCCAAAA AGGCCGGACG
 201  GGGCCTGCGG CGCACGGTCC TGGGAACGTC GTTGGCGCTG ACGCTGCTGG
 251  TTGGATATGT CTACGGGACG GACACCCGGG CGAGTGTGCA TCGGTACGGT
 301  GTTGTGCCGC TGATTAGAGC ATTGTATCCT GATGCGGAAG ATGCGCATCA
 351  TATTGGTGTC GATACTTTAA AGATGCTGTA TAAGTATGGT CTGCATCCAA
 401  GGGAACGGGG GGATCCGGAT GGAGATGGGG CGCTGGCGAC AGAGGTCTTT
 451  GGGTATACAC TGTCAAACCC AATTGGCATA TCGGGCGGCC TGGACAAGCA
 501  TGCTGAGATC CCTGATCCGC TGTTCGCGAT CGGTCCTGCC ATTGTCGAAG
 551  TCGGGGGTAC GACACCCTTA CCACAGGATG GTAACCCGCG TCCTCGCGAA
 601  TTCCGACTTC CATCACAGAG AGCGATGATA AACCGGTACG GCCTCAACTC
 651  CAAAGGCGCA GATCACATGG CAGCTATCTT GGAGCAACGA GTACGCGATT
 701  TTGCCTACGC AAACGGATTT GGGGCATACG ATGCGGCTAA GCAGCGTGTA
 751  TTGGACGGCG AAGCTGGTGT GCCACCAGGT AGTCTTCAGC CTGGTAAGCT
 801  TTTAGCTGTC CAAGTGGCAA AGAACAAGGC CACTCCTGAC GGCGACATTG
 851  AAGCCATCAA GCGCGACTAT GTGTATTGCG TGGACCGTGT GGCCAAATAC
 901  GCTGATATTC TTGTTGTGAA TGTATCGAGC CCCAACACAC CCGGTCTCCG
 951  TGACCTTCAA GCCACTGCCC CGCTCACAGC TATCTTGAGT GCTGTCGTTG
1001  GCGCGGCAAA GAGCGTGAAC CGCAAGACCA AACCATATGT TATGGTCAAG

1051  GTCAGTCCGG ATGAAGACTC AGATGAACAA GTCTCTGGTA TCTGCGACGC
1101  CGTCCGAGCA TCCGGTGTCG ACGGAGTGAT TGTCGGAAAC ACAACAAACC
1151  GTCGCCCCGA CCCTATACCC CAAGGTTACA CTCTTCCGGC CAAGGAGCAG
1201  GCAACGTTGA AAGAAACCGG CGGGTATTCA GGTCCACAGC TGTTCGATCG
1251  CACAGTGGCC CTTGTGGCTC GGTACCGCTC CATGCTGGAT GCGGAGTCGG
1301  AAACGGCCGG ATCCGCCAAG GATTCAGCAG CGACCATAGC GCAAACAGAG
1351  CCAGGCTCGG AAAACGTTCC TCCTGTGGAA GCGCCAAGCG GACTGCCGCG
1401  CAAAGTTATC TTCGCTTCGG GTGGTATCAC CAACGGGAAG CAGGCTCACG
1451  CTGTTTTAGA CACAGGGGCA TCTGTTGCCA TGATGTACAC CGGTGTGGTC
1501  TATGGTGGCG TCGGCACTGT CACTCGAGTG AAGCAAGAAC TTCGAACGGC
1551  GAAAAAGGAG
```

The amino acid sequence encoded by the preferred resistance gene is:

```
  1  MATNSFRKLT  FSGASRLGGC  RRLPLTCRQL  RFASDSGAAA  ATTKATAESA
 51  AESASINVKE  APKKAGRGLR  RTVLGTSLAL  TLLVGYVYGT  DTRASVHRYG
101  VVPLIRALYP  DAEDAHHIGV  DTLKMLYKYG  LHPRERGDPD  GDGALATEVF
151  GYTLSNPIGI  SGGLDKHAEI  PDPLFAIGPA  IVEVGGTTPL  PQDGNPRPRE
201  FRLPSQRAMI  NRYGLNSKGA  DHMAAILEQR  VRDFAYANGF  GAYDAAKQRV
251  LDGEAGVPPG  SLQPGKLLAV  QVAKNKATPD  GDIEAIKRDY  VYCVDRVAKY
301  ADILVVNVSS  PNTPGLRDLQ  ATAPLTAILS  AVVGAAKSVN  RKTKPYVMVK
351  VSPDEDSDEQ  VSGICDAVRA  SGVDGVIVGN  TTNRRPDPIP  QGYTLPAKEQ
401  ATLKETGGYS. GPQLFDRTVA  LVARYRSMLD  AESETAGSAK  DSAATIAQTE
451  PGSENVPPVE  APSGLPRKVI  FASGGITNGK  QAHAVLDTGA  SVAMMYTGVV
501  YGGVGTVTRV  KQELRTAKKE
```

Another mutation of the DHOD gene giving rise to CCFQ resistance changes the cytidyl residue at position 344 of the intron-less coding sequence and the intron-containing sequence to a thymidyl residue, thus changing amino acid 115 from alanine to valine. The DNA sequence of this resistance gene is:

```
   1 ATGGCTACGA ATTCTTTCCG AAAACTCACT TTTTCAGGAG CCTCCCGTCT
  51 GGGTGGTTGT CGCCGTCTCC CACTAACCTG CAGACAACTT CGATTCGCCT
 101 CCGACAGCGG AGCCGCAGCG GCAACTACAA AAGCAACGGC CGAATCAGCA
 151 GCCGAGTCAG CTAGTATAAA CGTCAAAGAG GCACCCAAAA AGGCCGGACG
 201 GGGCCTGCGG CGCACGGTCC TGGGAACGTC GTTGGCGCTG ACGCTGCTGG
 251 TTGGATATGT CTACGGGACG GACACCCGGG CGAGTGTGCA TCGGTACGGT
 301 GTTGTGCCGC TGATTAGAGC ATTGTATCCT GATGCGGAAG ATGTGCATCA
 351 TATTGGTGTC GATACTTTAA AGATGCTGTA TAAGTATGGT CTGCATCCAA
 401 GGGAACGGGG GGATCCGGAT GGAGATGGGG CGCTGGCGAC AGAGĞTGGGT
 451 GGTCTTTTAT TGTGATGGAA GTTAAGGCCC TAATACTTAT GGCGGATAĞG
 501 TCTTTGGGTA TACACTGTCA AACCCAATTG GCATATCGGG CGGCCTGGAC
 551 AAGCATGCTG AGATCCCTGA TCCGCTGTTC GCGATCGGTC CTGCCATTGT
 601 CGAAGTCGGG GGTACGACAC CCTTACCACA GGATGGTAAC CCGCGTCCTC
 651 GCGTATTCCG ACTTCCATCA CAGAGAGCGA TGATAAACCG GTACGGCCTC
 701 AACTCCAAAG GCGCAGATCA CATGGCAGCT ATCTTGGAGC AACGAGTACG
 751 CGATTTTGCC TACGCAAACG GATTTGGGGC ATACGATGCG GCTAAGCAGC
 801 GTGTATTGGA CGGCGAAGCT GGTGTGCCAC CAGGTAGTCT TCAGCCTGGT
 851 AAGCTTTTAG CTGTCCAAGT GGCAAAGAAC AAGGCCACTC CTGACGGCGA
 901 CATTGAAGCC ATCAAGCGCG ACTATGTGTA TTGCGTGGAC CGTGTGGCCA
 951 AATACGCTGA TATTCTTGTT GTGAATGTAT CGAGCCCCAA CACACCCGGT
1001 CTCCGTGACC TTCAAGCCAC TGCCCCGCTC ACAGCTATCT TGAGTGCTGT
1051 CGTTGGCGCG GCAAAGAGCG TGAACCGCAA GACCAAACCA TATGTTATGG
1101 TCAAGGTCAG TCCGGATGAA GACTCAGATG AACAAGTCTC TGGTATCTGC
1151 GACGCCGTCC GAGCATCCGG TGTCGACGGA GTGATTGTCG GAAACACAAC
1201 AAACCGTCGC CCCGACCCTA TACCCCAAGG TTACACTCTT CCGGCCAAGG
1251 AGCAGGCAAC GTTGAAAGAA ACCGGCGGGT ATTCAGGTCC ACAGCTGTTC
1301 GATCGCACAG TGGCCCTTGT GGCTCGGTAC CGCTCCATGC TGGATGCGGA
1351 GTCGGAAACG GCCGGATCCG CCAAGGATTC AGCAGCGACC ATAGCGCAAA
1401 CAGAGCCAGG CTCGGAAAAC GTTCCTCCTG TGGAAGCGCC AAGCGGACTG
1451 CCGCGCAAAG TTATCTTCGC TTCGGGTGGT ATCACCAACG GGAAGCAGGC
1501 TCACGCTGTT TTAGACACAG GGGCATCTGT TGCCATGATG TACACCGGTG
1551 TGGTCTATGG TGGCGTCGGC ACTGTCACTC GAGTGAAGCA AGAACTTCGA
1601 ACGGCGAAAA AGGAG
```

The coding sequence (with the intron removed) is:

```
   1  ATGGCTACGA ATTCTTTCCG AAAACTCACT TTTTCAGGAG CCTCCCGTCT
  51  GGGTGGTTGT CGCCGTCTCC CACTAACCTG CAGACAACTT CGATTCGCCT
 101  CCGACAGCGG AGCCGCAGCG GCAACTACAA AAGCAACGGC CGAATCAGCA
 151  GCCGAGTCAG CTAGTATAAA CGTCAAAGAG GCACCCAAAA AGGCCGGACG
 201  GGGCCTGCGG CGCACGGTCC TGGGAACGTC GTTGGCGCTG ACGCTGCTGG
 251  TTGGATATGT CTACGGGACG GACACCCGGG CGAGTGTGCA TCGGTACGGT
 301  GTTGTGCCGC TGATTAGAGC ATTGTATCCT GATGCGGAAG ATGTGCATCA
 351  TATTGGTGTC GATACTTTAA AGATGCTGTA TAAGTATGGT CTGCATCCAA
 401  GGGAACGGGG GGATCCGGAT GGAGATGGGG CGCTGGCGAC AGAGGTCTTT
 451  GGGTATACAC TGTCAAACCC AATTGGCATA TCGGGCGGCC TGGACAAGCA
 501  TGCTGAGATC CCTGATCCGC TGTTCGCGAT CGGTCCTGCC ATTGTCGAAG
 551  TCGGGGGTAC GACACCCTTA CCACAGGATG GTAACCCGCG TCCTCGCGTA
 601  TTCCGACTTC CATCACAGAG AGCGATGATA AACCGGTACG GCCTCAACTC
 651  CAAAGGCGCA GATCACATGG CAGCTATCTT GGAGCAACGA GTACGCGATT
 701  TTGCCTACGC AAACGGATTT GGGGCATACG ATGCGGCTAA GCAGCGTGTA
 751  TTGGACGGCG AAGCTGGTGT GCCACCAGGT AGTCTTCAGC CTGGTAAGCT
 801  TTTAGCTGTC CAAGTGGCAA AGAACAAGGC CACTCCTGAC GGCGACATTG
 851  AAGCCATCAA GCGCGACTAT GTGTATTGCG TGGACCGTGT GGCCAAATAC
 901  GCTGATATTC TTGTTGTGAA TGTATCGAGC CCCAACACAC CCGGTCTCCG
 951  TGACCTTCAA GCCACTGCCC CGCTCACAGC TATCTTGAGT GCTGTCGTTG
1001  GCGCGGCAAA GAGCGTGAAC CGCAAGACCA AACCATATGT TATGGTCAAG
1051  GTCAGTCCGG ATGAAGACTC AGATGAACAA GTCTCTGGTA TCTGCGACGC
1101  CGTCCGAGCA TCCGGTGTCG ACGGAGTGAT TGTCGGAAAC ACAACAAACC
1151  GTCGCCCCGA CCCTATACCC CAAGGTTACA CTCTTCCGGC CAAGGAGCAG
1201  GCAACGTTGA AAGAAACCGG CGGGTATTCA GGTCCACAGC TGTTCGATCG
1251  CACAGTGGCC CTTGTGGCTC GGTACCGCTC CATGCTGGAT GCGGAGTCGG
1301  AAACGGCCGG ATCCGCCAAG GATTCAGCAG CGACCATAGC GCAAACAGAG
1351  CCAGGCTCGG AAAACGTTCC TCCTGTGGAA GCGCCAAGCG GACTGCCGCG
1401  CAAAGTTATC TTCGCTTCGG GTGGTATCAC CAACGGGAAG CAGGCTCACG
1451  CTGTTTTAGA CACAGGGGCA TCTGTTGCCA TGATGTACAC CGGTGTGGTC
1501  TATGGTGGCG TCGGCACTGT CACTCGAGTG AAGCAAGAAC TTCGAACGGC
1551  GAAAAAGGAG
```

The amino acid sequence encoded by this resistance gene is:

```
  1  MATNSFRKLT  FSGASRLGGC  RRLPLTCRQL  RFASDSGAAA  ATTKATAESA
 51  AESASINVKE  APKKAGRGLR  RTVLGTSLAL  TLLVGYVYGT  DTRASVHRYG
101  VVPLIRALYP  DAEDVHHIGV  DTLKMLYKYG  LHPRERGDPD  GDGALATEVF
151  GYTLSNPIGI  SGGLDKHAEI  PDPLFAIGPA  IVEVGGTTPL  PQDGNPRPRV
201  FRLPSQRAMI  NRYGLNSKGA  DHMAAILEQR  VRDFAYANGF  GAYDAAKQRV
251  LDGEAGVPPG  SLQPGKLLAV  QVAKNKATPD  GDIEAIKRDY  VYCVDRVAKY
301  ADILVVNVSS  PNTPGLRDLQ  ATAPLTAILS  AVVGAAKSVN  RKTKPYVMVK
351  VSPDEDSDEQ  VSGICDAVRA  SGVDGVIVGN  TTNRRPDPIP  QGYTLPAKEQ
401  ATLKETGGYS  GPQLFDRTVA  LVARYRSMLD  AESETAGSAK  DSAATIAQTE
451  PGSENVPPVE  APSGLPRKVI  FASGGITNGK  QAHAVLDTGA  SVAMMYTGVV
501  YGGVGTVTRV  KQELRTAKKE
```

As those skilled in the art will recognize the present invention allows to generate other mutant DHOD genes at will. Given the DNA sequence for the DHOD gene, procedures familiar to skilled artisans are used to generate mutant DHOD enzymes that vary from the natural DHOD enzyme at any number of amino acid residue positions. Such mutant enzymes would be encoded by mutant DHOD coding sequences, including sequences in which amino acid codons have been deleted from or inserted into the natural DHOD coding sequence. Such mutant DNA sequences are within the scope of the present invention, because even if one cannot predict absolutely whether a given mutation will affect activity of the encoded DHOD, one may quickly use routine procedures to ascertain whether a mutation gives rise to a CCFQ resistance-conferring DNA sequence.

Furthermore, the present invention is not limited to isolated DNA sequences comprising an entire mutant DHOD gene. Because fungal recombinant DNA vectors can integrate into the fungal genome by homologous recombination, the only limitation on the resistance-conferring DNA sequence is that it be capable of conferring resistance to CCFQ when integrated into the wild-type DHOD gene. This event readily occurs even when only a portion of the DHOD gene is encoded by the recombinant DNA vector. See Examples, infra.

The mutant DHOD genes of the present invention are useful in any fungus which is sensitive to CCFQ and where the mechanism of action of CCFQ involves dihydroorotate dehydrogenase. Preferred fungi are Aspergillus, Penicillium, Cephalosporium and Neurospora. Preferred species of these fungi are Aspergillus nidulans, Penicillium chrysogenum, Cephalosporium acremonium and Neurospora crassa.

Those skilled in the art will recognize that the DNA sequences depicted above are an important part of the present invention. The above sequences can be conventionally synthesized by the modified phosphotriester method using fully protected deoxyribonucleotide building blocks. Such synthetic methods are well known in the art and can be carried out in substantial accordance with the procedure of Itakura et al., 1977, Science 198:1056 and Crea et al., 1978, Proc. Nat. Acad. Sci. USA 75:5765. In addition, an especially preferred method is disclosed in Hsiung et al., 1983, Nucleic Acid Research 11:3227 and Narang et al., 1980, Methods in Enzymology 68:90. In addition to the manual procedures referenced above, the DNA sequence can be synthesized using automated DNA synthesizers, such as the ABS (Applied Biosystems, 850 Lincoln Centre Drive, Foster City, CA 94404) 380B DNA Synthesizer. The DNA sequence can also be generated by the polymerase chain reaction. See, e.g., U.S. Patents Serial Nos. 4,800,159 and 4,683,202 and European Patent Publication No. 0258017, published March 2, 1987.

The amino acid sequences depicted above can be encoded by a multitude of different DNA sequences because most of the amino acid residues are encoded by more than one DNA triplet. Because these alternate DNA sequences would encode the same amino acid residue sequences of the present invention, the present invention further comprises these alternate sequences.

The present invention comprises DNA compounds and recombinant DNA cloning and expression vectors that comprise CCFQ resistance-conferring DNA sequences. The preferred CCFQ resistance-conferring sequences of the present invention were isolated from mutated strains of A. nidulans. A genomic library of the total genomic DNA of a mutated A. nidulans strain was constructed and examined for the presence of CCFQ resistanceconferring DNA sequences.

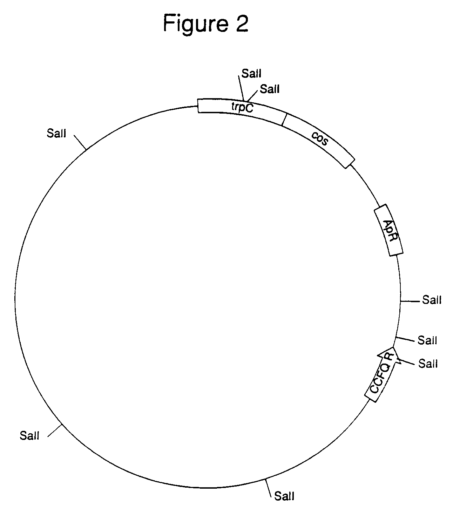

The preferred CCFQ resistance-conferring gene can be isolated on an ~8.3 kb partial SalI fragment derived from plasmid pGAR150, which has been deposited with the Northern Regional Research Laboratories (NRRL), Peoria, IL 61604, in E. coli K12 HB101 under the accession number NRRL B-18692 (date of deposit: July 30, 1990). A restriction site and function map of plasmid pGAR150 is presented in Figure 2 of the accompanying drawings. The ~7.3 kb Sall and ~5.4 kb HindIII restriction fragments of pGAR150 also confer resistance to CCFQ, but do not comprise the entire mutant DHOD gene.

Plasmid pGAR150 serves as useful starting material for the construction of other vectors of the invention. These vectors are especially useful in a method for constructing a recombinant host cell resistant to CCFQ, said method comprising transforming a host cell with a recombinant DNA vector that comprises an isolated DNA sequence which imparts resistance to CCFQ.

The following Examples are provided to further illustrate and exemplify, but not limit the scope of the present invention.

## Example 1

### Mutagenesis of Aspergillus nidulans

The growth medium for A. nidulans is 33.6 g malt extract agar (Difco Laboratories, P.O. Box 1058, Detroit, MI 48232) plus 6 g agar (Gibco Laboratories, P.O. Box 68, Grand Island, NY 14072) per liter of media. To grow A. nidulans FGSC610, add 1 ml of 1 mg/ml p-aminobenzoic acid (filter sterilized) per liter of media. To grow A. nidulans FGSC612, add 10 ml of 0.25 ml/ml riboflavin (filter sterilized) per liter of media.

### A. Mutagenesis of A. nidulans FGSC612 with MNNG

A. nidulans FGSC612 was obtained from the Fungal Genetic Stock Center (FGSC), (University of Kansas Medical Center, Kansas City, Kansas 66103). A filter sterilized 1 mg/ml solution of N-methyl-N′-nitro-N-nitrosoguanidine (MNNG, Sigma Chemical Co., P.O. Box 14508, St. Louis, MO 63178) was prepared in neutral phosphate buffer (NPB). NPB is 4 g NaCl, 0.1 g $MgSO_4.7H_2O$, 7 g $Na_2HPO_4$ and 3 g $KH_2PO_4$ (pH 7.2) per liter of water and is filter sterilized. Eighteen ml of NPB were placed into each of four 50 ml test tubes labelled A, B, C and D. To each the was added $10^8$ A. nidulans FGSC612 conidia in 1 ml Tween/saline (0.08% NaCl, 0.025% Tween 20). The tubes were placed at 37°C. Nineteen ml of MNNG were added to tubes A, B and C and 19 ml of NPB were added to tube D as a control. Tube A was removed after 30 minutes incubation at 37°C.

The spores were collected by centrifugation in a swinging bucket rotor at 16000 x g for 10 minutes. All but about 1-1.5 ml of the supernatant was drawn off. The remainder of the supernatant and the spores were transferred to a 1.5 ml Eppendorf tube. The contents of the tube were subjected to microcentrifugation for 5 minutes. All but about 100 μl of the supernatant was then drawn off. The spores were resuspended in 1 ml NPB. The spores were again subjected to microcentrifugation and washed twice more in NPB. The final spore pellet was resuspended in 200 μl NPB. After 45 minutes at 37°C, the contents of tube B were treated as above. After 60 minutes at 37°C, the contents of tubes C and D were subjected to the above treatment.

The spores from each tube were divided and spread on two plates of A. nidulans growth medium containing 2.5 μg/ml CCFQ. CCFQ was made according to procedures described in European Patent Publication No. 326330, published August 2, 1989. The plates were incubated at 37°C. Resistant colonies appeared in 3-4 days. The spores from tube D indicated the rate of spontaneously resistant mutants which was <1 per $10^8$ spores as compared to mutagenized A. nidulans, which was approximately 1 per $10^6$ spores. The A. nidulans mutant comprising the preferred CCFQ resistance gene was prepared as described above.

### B. Mutagenesis of A. nidulans FGSC610 with EMS

A. nidulans FGSC610 was obtained from the FGSC. Conidia ($10^8$) of A. nidulans in 1 ml Tween/saline were added to each of four test tubes, labelled A, B, C and D, containing 29 ml of NPB at 37°C. Ninety μl of methanesulfonic acid ethyl ester (EMS; Sigma Chemical Co., P.O. Box 14508, St. Louis, MO 63178) (final concentration of EMS = 0.3%) were added to tubes A, B and C and all tubes were incubated at 37°C. The remainder of the procedure was identical to Example 1A except that tube A was incubated for 30 minutes, tube B was incubated for 60 minutes and tubes C and D were incubated for 120 minutes. This procedure yielded a mutant comprising the resistance gene containing a thymidyl residue at position 344 of the dihydroorotate dehydrogenase gene.

## Example 2

### A. Preparation of Cosmid Libraries

Libraries were prepared from the DNA of mutants of Aspergillus nidulans constructed as described above.

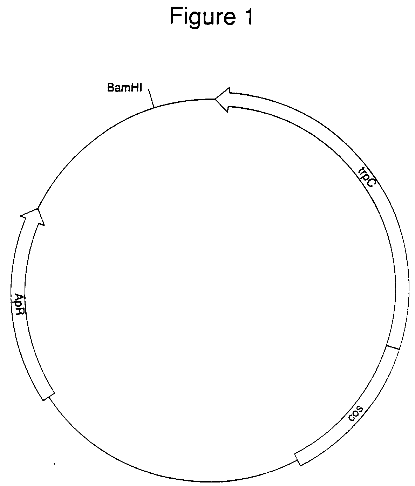

Chromosomal DNA was partially digested with restriction enzyme Sau3AI and inserted into BamHI-digested cosmid pKBY2. The insert size ranged from approximately 33-40 kilobase pairs. Cosmid pKBY2 is available from the ATCC under accession number 39898. A restriction site and function map of pKBY2 is provided in Figure 1. All of the procedures used in the preparation of the libraries are standard to skilled artisans.

B. Isolation of CCFQ Resistance Genes

Cosmids were combined into groups of 24 and analyzed for their ability to transform A. nidulans FGSC237 to resistance to 1.5 $\mu$g/ml CCFQ. A single cosmid was then isolated that also transformed A. nidulans FGSC237 to CCFQ resistance.

The A. nidulans transformations were carried out as described below.

C. A. nidulans Transformation Procedure

A 1-liter baffled flask containing 400 ml of glucose minimal media (see below) plus any necessary supplements was inoculated with $4 \times 10^8$ A. nidulans conidia. The culture was grown for approximately 15 hr at 30°C and 250 rpm. The stage of the culture is critical. The mycelium should be light and fluffy looking. Protoplasts derived from overgrown cultures (small mycelial balls) transform much less efficiently.

The mycelia were harvested by filtration through Miracloth (Calbiochem Corp., P.O. Box 12087, San Diego, CA 92112) and washed with 200 ml of sterile water. The mycelia were transferred to a 50 ml conical tube and resuspended in 40 ml of 1.2M MgSO4/10mM NaPO$_4$ (pH 5.8) by shaking and vortexing. Seventy-five mg of Novozyme 234 (Novo Nordisk Biolabs, Inc.) and 0.75 ml of beta-glucuronidase (Sigma Chemical Co.) were added to the mycelia and the mixture was incubated with slow rotation at 27°C for 1.5-2.0 hr. The amount of Novozyme required can vary with the batch obtained from the supplier and the incubation time can vary between experiments. Protoplasting was monitored microscopically at timed intervals to insure optimum results. Vortexing the protoplast solution for a few seconds can reduce clumping and improve protoplast yield. Equal volumes of the protoplast solution were transferred into two 30 ml Sarstedt tubes and carefully overlayed with 8 ml of sterile ST buffer (ST = 0.6M sorbitol/0.1M Tris-HCl, pH 7.0). The tubes were centrifuged at 4,000 X g for 15 min at 20°C in a swinging bucket rotor. Protoplasts were harvested from the interface and washed two times in 4 volumes of STC buffer (STC = 1.2M sorbitol/10mM CaCl$_2$/10mM Tris-HCl, pH 7.5). After each wash the protoplasts were recovered by centrifugation at 1,500 X g for 10 min at 20°C in a swinging bucket rotor. The protoplasts were resuspended in 1 ml of STC, counted in a haemocytometer and adjusted to a final concentration of $2 \times 10^8$/ml. For transformation, 2-5 $\mu$g of DNA in less than 10 $\mu$l of water was added to 150 $\mu$l ($3 \times 10^7$) protoplasts in a 1.5 ml Eppendorf tube. DNA and protoplasts were mixed and incubated at room temperature for 15 min. One ml of a polyethyleneglycol (PEG) solution (see PTC below) was then added and the solutions were thoroughly mixed and allowed to stand at room temperature for 15 min. The tubes were centrifuged for 4 min and as much as possible of the PEG solution was removed from the protoplast pellet. Protoplasts were resuspended in 1 ml of sterile YGS media (YGS = 0.5% yeast extract/ 2.0% glucose/1.2M sorbitol) containing any supplements necessary for fungal growth and incubated at 27°C for 2-3 hr with slow rotation. Cells were harvested by centrifugation for 3 min and resuspended in 150 $\mu$l of STC. 50 $\mu$l of this suspension were used to inoculate a single petri plate. The media on which the transformed fungi were selected consisted of glucose minimal media containing 1.2M sorbitol, any supplements that a transformed cell would need for growth (e.g., p-aminobenzoic acid was added to the media for transformations of strain FGSC237 with pKBY2-based vectors, but tryptophan was not added because any FGSC237 cell transformed with a cosmid containing pKBY2 should no longer require tryptophan) and 1.5 $\mu$g/ml CCFQ.

Plates were incubated at 37°C for 3-5 days. For transformations that resulted in very few CCFQ resistant colonies, spores from those colonies were replated on media containing CCFQ to insure that the resistance trait was stable. PTC solution is 60% polyethylene glycol (British Drug Houses)/4000 10mM CaCl$_2$/10mM Tris-HCl, pH 7.5). The solution was filter sterilized and stored at -20°C in 5-10 ml aliquots.

Glucose minimal media (liquid)

## Trace Elements Solution

| | |
|---|---|
| $FeSO_4 \cdot 7H_2O$ | 1.00 g/liter |
| $ZnSO_4 \cdot 7H_2O$ | 8.80 g/liter |
| $CuSO_4 \cdot 5H_2O$ | 0.40 g/liter |
| $MnSO_4 \cdot 4H_2O$ | 0.15 g/liter |
| $Na_2B_4O_7 \cdot 10H_2O$ | 0.10 g/liter |
| $(NH_4)_6Mo_7O_{24} \cdot 4H_2O$ | 0.05 g/liter |

## Stock Salts Solution

| | |
|---|---|
| $NaNO_3$ | 60.0 g/liter |
| $KCl$ | 5.2 g/liter |
| $KH_2PO_4$ | 15.2 g/liter |
| Trace elements solution | 10.0 ml/liter |

Adjust pH to 6.5

To make 200 ml of glucose minimal medium combine 40 ml of stock salt solution with 140 ml of $H_2O$ and autoclave. Then add 10 ml of filter sterilized 20% glucose and 10 ml of filter sterilized 2% $MgSO_4 \cdot 7H_2O$. Add p-aminobenzoic acid (paba), riboflavin, etc. as needed depending on the auxotrophic requirements of the <u>A</u>. nidulans strain being grown. For 250 ml of solid minimal media combine 25.0 ml of stock salts, 10 ml 20% glucose, 10 ml 2% $MgSO4 \cdot 7H_2O$, 3.75g agar and 205 ml $H_2O$. Autoclave to sterilize.

Example 3

Analysis of CCFQ Resistance Genes

A portion of the resistance gene isolated above was used as a probe to isolate a resistance gene from the library derived from <u>A</u>. nidulans treated with MNNG. This resulted in the isolation of the preferred resistance gene of the invention. A cosmid comprising this gene is called pGAR150 and can be obtained from the NRRL (Northern Regional Research Center (NRRL), Agricultural Research Service, U.S. Department of Agriculture, Peoria, Illinois, 61604) in <u>E</u>. <u>coli</u> K12 HB101 under accession number NRRL B-18692 (date of deposit: July 30, 1990).

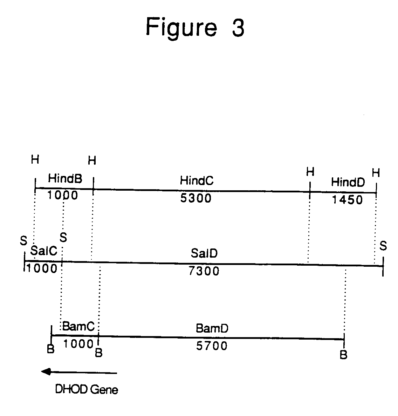

A restriction site and function map is presented in Figure 2. A more detailed restriction map of the DHOD gene region is presented in Figure 3.

Various restriction fragments derived from cosmids comprising resistance genes were cloned into pUC19 (Bethesda Research Laboratories, Inc., P.O. Box 577, Gaithersburg, MD 20760) and tested for their ability to transform <u>A</u>. nidulans to CCFQ resistance. The transformations were carried out as described above. <u>See</u> Figure 3 for an explanation of each fragment and placement of the gene.

EP 0 471 466 A2

## Table I

| DNA Sample<br>EMS treated DNA | CCFQ-resistant<br>transformants/μg DNA |
|---|---|
| Entire cosmid | 35 |
| SalD | 6 |
| HindC | 3 |
| HindD | 0 |
| BamD | 1 |
| **MNNG treated-DNA** | |
| Entire cosmid | 60 |
| SalD | 8 |
| HindC | 6 |
| HindD | 0 |
| BamD | 0 |
| Wild-type gene containing cosmid | 0 |
| pUC19 | 0 |
| pKBY2 | 0 |

These results demonstrate that the entire open reading frame of the gene is not required to transform to CCFQ resistance.

## Claims

1. A DNA compound comprising an isolated DNA sequence encoding dihydroorotate dehydrogenase activity of Aspergillus nidulans.

2. The DNA compound of Claim 1 comprising an isolated DNA sequence encoding the amino acid sequence

```
  1  MATNSFRKLT FSGASRLGGC RRLPLTCRQL RFASDSGAAA ATTKATAESA
 51  AESASINVKE APKKAGRGLR RTVLGTSLAL TLLVGYVYGT DTRASVHRYG
101  VVPLIRALYP DAEDAHHIGV DTLKMLYKYG LHPRERGDPD GDGALATEVF
151  GYTLSNPIGI SGGLDKHAEI PDPLFAIGPA IVEVGGTTPL PQDGNPRPRV
201  FRLPSQRAMI NRYGLNSKGA DHMAAILEQR VRDFAYANGF GAYDAAKQRV
251  LDGEAGVPPG SLQPGKLLAV QVAKNKATPD GDIEAIKRDY VYCVDRVAKY
301  ADILVVNVSS PNTPGLRDLQ ATAPLTAILS AVVGAAKSVN RKTKPYVMVK
351  VSPDEDSDEQ VSGICDAVRA SGVDGVIVGN TTNRRPDPIP QGYTLPAKEQ
401  ATLKETGGYS GPQLFDRTVA LVARYRSMLD AESETAGSAK DSAATIAQTE
451  PGSENVPPVE APSGLPRKVI FASGGITNGK QAHAVLDTGA SVAMMYTGVV
501  YGGVGTVTRV KQELRTAKKE.
```

3. The DNA compound of Claim 2 comprising an isolated DNA sequence

15

```
   1 ATGGCTACGA ATTCTTTCCG AAAACTCACT TTTTCAGGAG CCTCCCGTCT
  51 GGGTGGTTGT CGCCGTCTCC CACTAACCTG CAGACAACTT CGATTCGCCT
 101 CCGACAGCGG AGCCGCAGCG GCAACTACAA AAGCAACGGC CGAATCAGCA
 151 GCCGAGTCAG CTAGTATAAA CGTCAAAGAG GCACCCAAAA AGGCCGGACG
 201 GGGCCTGCGG CGCACGGTCC TGGGAACGTC GTTGGCGCTG ACGCTGCTGG
 251 TTGGATATGT CTACGGGACG GACACCCGGG CGAGTGTGCA TCGGTACGGT
 301 GTTGTGCCGC TGATTAGAGC ATTGTATCCT GATGCGGAAG ATGCGCATCA
 351 TATTGGTGTC GATACTTTAA AGATGCTGTA TAAGTATGGT CTGCATCCAA
 401 GGGAACGGGG GGATCCGGAT GGAGATGGGG CGCTGGCGAC AGAGGTGGGT
 451 GGTCTTTTAT TGTGATGGAA GTTAAGGCCC TAATACTTAT GGCGGATAGG

 501 TCTTTGGGTA TACACTGTCA AACCCAATTG GCATATCGGG CGGCCTGGAC
 551 AAGCATGCTG AGATCCCTGA TCCGCTGTTC GCGATCGGTC CTGCCATTGT
 601 CGAAGTCGGG GGTACGACAC CCTTACCACA GGATGGTAAC CCGCGTCCTC
 651 GCGTATTCCG ACTTCCATCA CAGAGAGCGA TGATAAACCG GTACGGCCTC
 701 AACTCCAAAG GCGCAGATCA CATGGCAGCT ATCTTGGAGC AACGAGTACG
 751 CGATTTTGCC TACGCAAACG GATTTGGGGC ATACGATGCG GCTAAGCAGC
 801 GTGTATTGGA CGGCGAAGCT GGTGTGCCAC CAGGTAGTCT TCAGCCTGGT
 851 AAGCTTTTAG CTGTCCAAGT GGCAAAGAAC AAGGCCACTC CTGACGGCGA
 901 CATTGAAGCC ATCAAGCGCG ACTATGTGTA TTGCGTGGAC CGTGTGGCCA
 951 AATACGCTGA TATTCTTGTT GTGAATGTAT CGAGCCCCAA CACACCCGGT
1001 CTCCGTGACC TTCAAGCCAC TGCCCCGCTC ACAGCTATCT TGAGTGCTGT
1051 CGTTGGCGCG GCAAAGAGCG TGAACCGCAA GACCAAACCA TATGTTATGG
1101 TCAAGGTCAG TCCGGATGAA GACTCAGATG AACAAGTCTC TGGTATCTGC
1151 GACGCCGTCC GAGCATCCGG TGTCGACGGA GTGATTGTCG GAAACACAAC
1201 AAACCGTCGC CCCGACCCTA TACCCCAAGG TTACACTCTT CCGGCCAAGG
1251 AGCAGGCAAC GTTGAAAGAA ACCGGCGGGT ATTCAGGTCC ACAGCTGTTC
1301 GATCGCACAG TGGCCCTTGT GGCTCGGTAC CGCTCCATGC TGGATGCGGA
1351 GTCGGAAACG GCCGGATCCG CCAAGGATTC AGCAGCGACC ATAGCGCAAA
1401 CAGAGCCAGG CTCGGAAAAC GTTCCTCCTG TGGAAGCGCC AAGCGGACTG
1451 CCGCGCAAAG TTATCTTCGC TTCGGGTGGT ATCACCAACG GGAAGCAGGC
1501 TCACGCTGTT TTAGACACAG GGGCATCTGT TGCCATGATG TACACCGGTG
1551 TGGTCTATGG TGGCGTCGGC ACTGTCACTC GAGTGAAGCA AGAACTTCGA
1601 ACGGCGAAAA AGGAG.
```

4.   The DNA compound of Claim 2 comprising an isolated DNA sequence

```
   1  ATGGCTACGA ATTCTTTCCG AAAACTCACT TTTTCAGGAG CCTCCCGTCT
  51  GGGTGGTTGT CGCCGTCTCC CACTAACCTG CAGACAACTT CGATTCGCCT
 101  CCGACAGCGG AGCCGCAGCG GCAACTACAA AAGCAACGGC CGAATCAGCA
 151  GCCGAGTCAG CTAGTATAAA CGTCAAAGAG GCACCCAAAA AGGCCGGACG
 201  GGGCCTGCGG CGCACGGTCC TGGGAACGTC GTTGGCGCTG ACGCTGCTGG
```

```
 251  TTGGATATGT CTACGGGACG GACACCCGGG CGAGTGTGCA TCGGTACGGT
 301  GTTGTGCCGC TGATTAGAGC ATTGTATCCT GATGCGGAAG ATGCGCATCA
 351  TATTGGTGTC GATACTTTAA AGATGCTGTA TAAGTATGGT CTGCATCCAA
 401  GGGAACGGGG GGATCCGGAT GGAGATGGGG CGCTGGCGAC AGAGGTCTTT
 451  GGGTATACAC TGTCAAACCC AATTGGCATA TCGGGCGGCC TGGACAAGCA
 501  TGCTGAGATC CCTGATCCGC TGTTCGCGAT CGGTCCTGCC ATTGTCGAAG
 551  TCGGGGGTAC GACACCCTTA CCACAGGATG GTAACCCGCG TCCTCGCGTA
 601  TTCCGACTTC CATCACAGAG AGCGATGATA AACCGGTACG GCCTCAACTC
 651  CAAAGGCGCA GATCACATGG CAGCTATCTT GGAGCAACGA GTACGCGATT
 701  TTGCCTACGC AAACGGATTT GGGGCATACG ATGCGGCTAA GCAGCGTGTA
 751  TTGGACGGCG AAGCTGGTGT GCCACCAGGT AGTCTTCAGC CTGGTAAGCT
 801  TTTAGCTGTC CAAGTGGCAA AGAACAAGGC CACTCCTGAC GGCGACATTG
 851  AAGCCATCAA GCGCGACTAT GTGTATTGCG TGGACCGTGT GGCCAAATAC
 901  GCTGATATTC TTGTTGTGAA TGTATCGAGC CCCAACACAC CCGGTCTCCG
 951  TGACCTTCAA GCCACTGCCC CGCTCACAGC TATCTTGAGT GCTGTCGTTG
1001  GCGCGGCAAA GAGCGTGAAC CGCAAGACCA AACCATATGT TATGGTCAAG
1051  GTCAGTCCGG ATGAAGACTC AGATGAACAA GTCTCTGGTA TCTGCGACGC
1101  CGTCCGAGCA TCCGGTGTCG ACGGAGTGAT TGTCGGAAAC ACAACAAACC
1151  GTCGCCCCGA CCCTATACCC CAAGGTTACA CTCTTCCGGC CAAGGAGCAG
1201  GCAACGTTGA AAGAAACCGG CGGGTATTCA GGTCCACAGC TGTTCGATCG
1251  CACAGTGGCC CTTGTGGCTC GGTACCGCTC CATGCTGGAT GCGGAGTCGG
1301  AAACGGCCGG ATCCGCCAAG GATTCAGCAG CGACCATAGC GCAAACAGAG
1351  CCAGGCTCGG AAAACGTTCC TCCTGTGGAA GCGCCAAGCG GACTGCCGCG
1401  CAAAGTTATC TTCGCTTCGG GTGGTATCAC CAACGGGAAG CAGGCTCACG
1451  CTGTTTTAGA CACAGGGGCA TCTGTTGCCA TGATGTACAC CGGTGTGGTC
1501  TATGGTGGCG TCGGCACTGT CACTCGAGTG AAGCAAGAAC TTCGAACGGC
1551  GAAAAAGGAG.
```

5. A DNA compound comprising an isolated DNA sequence encoding an 8-chloro-4-(2-chloro-4-fluorophenoxy)quinoline resistance gene.

6. The DNA compound of Claim 5 comprising an isolated DNA sequence encoding the amino acid sequence

```
  1   MATNSFRKLT FSGASRLGGC RRLPLTCRQL RFASDSGAAA ATTKATAESA
 51   AESASINVKE APKKAGRGLR RTVLGTSLAL TLLVGYVYGT DTRASVHRYG
101   VVPLIRALYP DAEDAHHIGV DTLKMLYKYG LHPRERGDPD GDGALATEVF
151   GYTLSNPIGI SGGLDKHAEI PDPLFAIGPA IVEVGGTTPL PQDGNPRPRE
201   FRLPSQRAMI NRYGLNSKGA DHMAAILEQR VRDFAYANGF GAYDAAKQRV
251   LDGEAGVPPG SLQPGKLLAV QVAKNKATPD GDIEAIKRDY VYCVDRVAKY
301   ADILVVNVSS PNTPGLRDLQ ATAPLTAILS AVVGAAKSVN RKTKPYVMVK
351   VSPDEDSDEQ VSGICDAVRA SGVDGVIVGN TTNRRPDPIP QGYTLPAKEQ
401   ATLKETGGYS GPQLFDRTVA LVARYRSMLD AESETAGSAK DSAATIAQTE
451   PGSENVPPVE APSGLPRKVI FASGGITNGK QAHAVLDTGA SVAMMYTGVV
501   YGGVGTVTRV KQELRTAKKE.
```

7.    The DNA compound of Claim 6 comprising an isolated DNA sequence

```
  1   ATGGCTACGA ATTCTTTCCG AAAACTCACT TTTTCAGGAG CCTCCCGTCT
 51   GGGTGGTTGT CGCCGTCTCC CACTAACCTG CAGACAACTT CGATTCGCCT
101   CCGACAGCGG AGCCGCAGCG GCAACTACAA AAGCAACGGC CGAATCAGCA
151   GCCGAGTCAG CTAGTATAAA CGTCAAAGAG GCACCCAAAA AGGCCGGACG
201   GGGCCTGCGG CGCACGGTCC TGGGAACGTC GTTGGCGCTG ACGCTGCTGG
251   TTGGATATGT CTACGGGACG GACACCCGGG CGAGTGTGCA TCGGTACGGT
301   GTTGTGCCGC TGATTAGAGC ATTGTATCCT GATGCGGAAG ATGCGCATCA
351   TATTGGTGTC GATACTTTAA AGATGCTGTA TAAGTATGGT CTGCATCCAA
401   GGGAACGGGG GGATCCGGAT GGAGATGGGG CGCTGGCGAC AGAGGTGGGT
451   GGTCTTTTAT TGTGATGGAA GTTAAGGCCC TAATACTTAT GGCGGATAGG
501   TCTTTGGGTA TACACTGTCA AACCCAATTG GCATATCGGG CGGCCTGGAC
```

```
 551 AAGCATGCTG AGATCCCTGA TCCGCTGTTC GCGATCGGTC CTGCCATTGT
 601 CGAAGTCGGG GGTACGACAC CCTTACCACA GGATGGTAAC CCGCGTCCTC
 651 GCGAATTCCG ACTTCCATCA CAGAGAGCGA TGATAAACCG GTACGGCCTC
 701 AACTCCAAAG GCGCAGATCA CATGGCAGCT ATCTTGGAGC AACGAGTACG
 751 CGATTTTGCC TACGCAAACG GATTTGGGGC ATACGATGCG GCTAAGCAGC
 801 GTGTATTGGA CGGCGAAGCT GGTGTGCCAC CAGGTAGTCT TCAGCCTGGT
 851 AAGCTTTTAG CTGTCCAAGT GGCAAAGAAC AAGGCCACTC CTGACGGCGA
 901 CATTGAAGCC ATCAAGCGCG ACTATGTGTA TTGCGTGGAC CGTGTGGCCA
 951 AATACGCTGA TATTCTTGTT GTGAATGTAT CGAGCCCCAA CACACCCGGT
1001 CTCCGTGACC TTCAAGCCAC TGCCCCGCTC ACAGCTATCT TGAGTGCTGT
1051 CGTTGGCGCG GCAAAGAGCG TGAACCGCAA GACCAAACCA TATGTTATGG
1101 TCAAGGTCAG TCCGGATGAA GACTCAGATG AACAAGTCTC TGGTATCTGC
1151 GACGCCGTCC GAGCATCCGG TGTCGACGGA GTGATTGTCG GAAACACAAC
1201 AAACCGTCGC CCCGACCCTA TACCCCAAGG TTACACTCTT CCGGCCAAGG
1251 AGCAGGCAAC GTTGAAAGAA ACCGGCGGGT ATTCAGGTCC ACAGCTGTTC
1301 GATCGCACAG TGGCCCTTGT GGCTCGGTAC CGCTCCATGC TGGATGCGGA
1351 GTCGGAAACG GCCGGATCCG CCAAGGATTC AGCAGCGACC ATAGCGCAAA
1401 CAGAGCCAGG CTCGGAAAAC GTTCCTCCTG TGGAAGCGCC AAGCGGACTG
1451 CCGCGCAAAG TTATCTTCGC TTCGGGTGGT ATCACCAACG GGAAGCAGGC
1501 TCACGCTGTT TTAGACACAG GGGCATCTGT TGCCATGATG TACACCGGTG
1551 TGGTCTATGG TGGCGTCGGC ACTGTCACTC GAGTGAAGCA AGAACTTCGA
1601 ACGGCGAAAA AGGAG.
```

8.   The DNA compound of Claim 6 comprising an isolated DNA sequence

```
  1 ATGGCTACGA ATTCTTTCCG AAAACTCACT TTTTCAGGAG CCTCCCGTCT
 51 GGGTGGTTGT CGCCGTCTCC CACTAACCTG CAGACAACTT CGATTCGCCT
101 CCGACAGCGG AGCCGCAGCG GCAACTACAA AAGCAACGGC CGAATCAGCA
151 GCCGAGTCAG CTAGTATAAA CGTCAAAGAG GCACCCAAAA AGGCCGGACG
201 GGGCCTGCGG CGCACGGTCC TGGGAACGTC GTTGGCGCTG ACGCTGCTGG
251 TTGGATATGT CTACGGGACG GACACCCGGG CGAGTGTGCA TCGGTACGGT
```

```
 301  GTTGTGCCGC TGATTAGAGC ATTGTATCCT GATGCGGAAG ATGCGCATCA
 351  TATTGGTGTC GATACTTTAA AGATGCTGTA TAAGTATGGT CTGCATCCAA
 401  GGGAACGGGG GGATCCGGAT GGAGATGGGG CGCTGGCGAC AGAGGTCTTT
 451  GGGTATACAC TGTCAAACCC AATTGGCATA TCGGGCGGCC TGGACAAGCA
 501  TGCTGAGATC CCTGATCCGC TGTTCGCGAT CGGTCCTGCC ATTGTCGAAG
 551  TCGGGGGTAC GACACCCTTA CCACAGGATG GTAACCCGCG TCCTCGCGAA
 601  TTCCGACTTC CATCACAGAG AGCGATGATA AACCGGTACG GCCTCAACTC
 651  CAAAGGCGCA GATCACATGG CAGCTATCTT GGAGCAACGA GTACGCGATT
 701  TTGCCTACGC AAACGGATTT GGGGCATACG ATGCGGCTAA GCAGCGTGTA
 751  TTGGACGGCG AAGCTGGTGT GCCACCAGGT AGTCTTCAGC CTGGTAAGCT
 801  TTTAGCTGTC CAAGTGGCAA AGAACAAGGC CACTCCTGAC GGCGACATTG
 851  AAGCCATCAA GCGCGACTAT GTGTATTGCG TGGACCGTGT GGCCAAATAC
 901  GCTGATATTC TTGTTGTGAA TGTATCGAGC CCCAACACAC CCGGTCTCCG
 951  TGACCTTCAA GCCACTGCCC CGCTCACAGC TATCTTGAGT GCTGTCGTTG
1001  GCGCGGCAAA GAGCGTGAAC CGCAAGACCA AACCATATGT TATGGTCAAG
1051  GTCAGTCCGG ATGAAGACTC AGATGAACAA GTCTCTGGTA TCTGCGACGC
1101  CGTCCGAGCA TCCGGTGTCG ACGGAGTGAT TGTCGGAAAC ACAACAAACC
1151  GTCGCCCCGA CCCTATACCC CAAGGTTACA CTCTTCCGGC CAAGGAGCAG
1201  GCAACGTTGA AAGAAACCGG CGGGTATTCA GGTCCACAGC TGTTCGATCG
1251  CACAGTGGCC CTTGTGGCTC GGTACCGCTC CATGCTGGAT GCGGAGTCGG
1301  AAACGGCCGG ATCCGCCAAG GATTCAGCAG CGACCATAGC GCAAACAGAG
1351  CCAGGCTCGG AAAACGTTCC TCCTGTGGAA GCGCCAAGCG GACTGCCGCG
1401  CAAAGTTATC TTCGCTTCGG GTGGTATCAC CAACGGGAAG CAGGCTCACG
1451  CTGTTTTAGA CACAGGGGCA TCTGTTGCCA TGATGTACAC CGGTGTGGTC
1501  TATGGTGGCG TCGGCACTGT CACTCGAGTG AAGCAAGAAC TTCGAACGGC
1551  GAAAAAGGAG.
```

9.    The DNA compound of Claim 5 comprising an isolated DNA sequence encoding the amino acid sequence

```
  1  MATNSFRKLT FSGASRLGGC RRLPLTCRQL RFASDSGAAA ATTKATAESA
 51  AESASINVKE APKKAGRGLR RTVLGTSLAL TLLVGYVYGT DTRASVHRYG
101  VVPLIRALYP DAEDVHHIGV DTLKMLYKYG LHPRERGDPD GDGALATEVF
151  GYTLSNPIGI SGGLDKHAEI PDPLFAIGPA IVEVGGTTPL PQDGNPRPRV
201  FRLPSQRAMI NRYGLNSKGA DHMAAILEQR VRDFAYANGF GAYDAAKQRV
251  LDGEAGVPPG SLQPGKLLAV QVAKNKATPD GDIEAIKRDY VYCVDRVAKY
301  ADILVVNVSS PNTPGLRDLQ ATAPLTAILS AVVGAAKSVN RKTKPYVMVK
351  VSPDEDSDEQ VSGICDAVRA SGVDGVIVGN TTNRRPDPIP QGYTLPAKEQ
401  ATLKETGGYS GPQLFDRTVA LVARYRSMLD AESETAGSAK DSAATIAQTE
451  PGSENVPPVE APSGLPRKVI FASGGITNGK QAHAVLDTGA SVAMMYTGVV
501  YGGVGTVTRV KQELRTAKKE.
```

10. The DNA compound of Claim 9 comprising an isolated DNA sequence

```
   1 ATGGCTACGA ATTCTTTCCG AAAACTCACT TTTTCAGGAG CCTCCCGTCT
  51 GGGTGGTTGT CGCCGTCTCC CACTAACCTG CAGACAACTT CGATTCGCCT
 101 CCGACAGCGG AGCCGCAGCG GCAACTACAA AAGCAACGGC CGAATCAGCA
 151 GCCGAGTCAG CTAGTATAAA CGTCAAAGAG GCACCCAAAA AGGCCGGACG
 201 GGGCCTGCGG CGCACGGTCC TGGGAACGTC GTTGGCGCTG ACGCTGCTGG
 251 TTGGATATGT CTACGGGACG GACACCCGGG CGAGTGTGCA TCGGTACGGT
 301 GTTGTGCCGC TGATTAGAGC ATTGTATCCT GATGCGGAAG ATGTGCATCA
 351 TATTGGTGTC GATACTTTAA AGATGCTGTA TAAGTATGGT CTGCATCCAA
 401 GGGAACGGGG GGATCCGGAT GGAGATGGGG CGCTGGCGAC AGAGGTGGGT
 451 GGTCTTTTAT TGTGATGGAA GTTAAGGCCC TAATACTTAT GGCGGATAGG
 501 TCTTTGGGTA TACACTGTCA AACCCAATTG GCATATCGGG CGGCCTGGAC
 551 AAGCATGCTG AGATCCCTGA TCCGCTGTTC GCGATCGGTC CTGCCATTGT
 601 CGAAGTCGGG GGTACGACAC CCTTACCACA GGATGGTAAC CCGCGTCCTC
 651 GCGTATTCCG ACTTCCATCA CAGAGAGCGA TGATAAACCG GTACGGCCTC

 701 AACTCCAAAG GCGCAGATCA CATGGCAGCT ATCTTGGAGC AACGAGTACG
 751 CGATTTTGCC TACGCAAACG GATTTGGGGC ATACGATGCG GCTAAGCAGC
 801 GTGTATTGGA CGGCGAAGCT GGTGTGCCAC CAGGTAGTCT TCAGCCTGGT
 851 AAGCTTTTAG CTGTCCAAGT GGCAAAGAAC AAGGCCACTC CTGACGGCGA
 901 CATTGAAGCC ATCAAGCGCG ACTATGTGTA TTGCGTGGAC CGTGTGGCCA
 951 AATACGCTGA TATTCTTGTT GTGAATGTAT CGAGCCCCAA CACACCCGGT
1001 CTCCGTGACC TTCAAGCCAC TGCCCCGCTC ACAGCTATCT TGAGTGCTGT
1051 CGTTGGCGCG GCAAAGAGCG TGAACCGCAA GACCAAACCA TATGTTATGG
1101 TCAAGGTCAG TCCGGATGAA GACTCAGATG AACAAGTCTC TGGTATCTGC
1151 GACGCCGTCC GAGCATCCGG TGTCGACGGA GTGATTGTCG GAAACACAAC
1201 AAACCGTCGC CCCGACCCTA TACCCCAAGG TTACACTCTT CCGGCCAAGG
1251 AGCAGGCAAC GTTGAAAGAA ACCGGCGGGT ATTCAGGTCC ACAGCTGTTC
1301 GATCGCACAG TGGCCCTTGT GGCTCGGTAC CGCTCCATGC TGGATGCGGA
1351 GTCGGAAACG GCCGGATCCG CCAAGGATTC AGCAGCGACC ATAGCGCAAA
1401 CAGAGCCAGG CTCGGAAAAC GTTCCTCCTG TGGAAGCGCC AAGCGGACTG
1451 CCGCGCAAAG TTATCTTCGC TTCGGGTGGT ATCACCAACG GGAAGCAGGC
1501 TCACGCTGTT TTAGACACAG GGGCATCTGT TGCCATGATG TACACCGGTG
1551 TGGTCTATGG TGGCGTCGGC ACTGTCACTC GAGTGAAGCA AGAACTTCGA
1601 ACGGCGAAAA AGGAG.
```

11. The DNA compound of Claim 9 comprising an isolated DNA sequence

```
   1  ATGGCTACGA ATTCTTTCCG AAAACTCACT TTTTCAGGAG CCTCCCGTCT
  51  GGGTGGTTGT CGCCGTCTCC CACTAACCTG CAGACAACTT CGATTCGCCT
 101  CCGACAGCGG AGCCGCAGCG GCAACTACAA AAGCAACGGC CGAATCAGCA
 151  GCCGAGTCAG CTAGTATAAA CGTCAAAGAG GCACCCAAAA AGGCCGGACG
 201  GGGCCTGCGG CGCACGGTCC TGGGAACGTC GTTGGCGCTG ACGCTGCTGG
 251  TTGGATATGT CTACGGGACG GACACCCGGG CGAGTGTGCA TCGGTACGGT
 301  GTTGTGCCGC TGATTAGAGC ATTGTATCCT GATGCGGAAG ATGTGCATCA
 351  TATTGGTGTC GATACTTTAA AGATGCTGTA TAAGTATGGT CTGCATCCAA
 401  GGGAACGGGG GGATCCGGAT GGAGATGGGG CGCTGGCGAC AGAGGTCTTT

 451  GGGTATACAC TGTCAAACCC AATTGGCATA TCGGGCGGCC TGGACAAGCA
 501  TGCTGAGATC CCTGATCCGC TGTTCGCGAT CGGTCCTGCC ATTGTCGAAG
 551  TCGGGGGTAC GACACCCTTA CCACAGGATG GTAACCCGCG TCCTCGCGTA
 601  TTCCGACTTC CATCACAGAG AGCGATGATA AACCGGTACG GCCTCAACTC
 651  CAAAGGCGCA GATCACATGG CAGCTATCTT GGAGCAACGA GTACGCGATT
 701  TTGCCTACGC AAACGGATTT GGGGCATACG ATGCGGCTAA GCAGCGTGTA
 751  TTGGACGGCG AAGCTGGTGT GCCACCAGGT AGTCTTCAGC CTGGTAAGCT
 801  TTTAGCTGTC CAAGTGGCAA AGAACAAGGC CACTCCTGAC GGCGACATTG
 851  AAGCCATCAA GCGCGACTAT GTGTATTGCG TGGACCGTGT GGCCAAATAC
 901  GCTGATATTC TTGTTGTGAA TGTATCGAGC CCCAACACAC CCGGTCTCCG
 951  TGACCTTCAA GCCACTGCCC CGCTCACAGC TATCTTGAGT GCTGTCGTTG
1001  GCGCGGCAAA GAGCGTGAAC CGCAAGACCA AACCATATGT TATGGTCAAG
1051  GTCAGTCCGG ATGAAGACTC AGATGAACAA GTCTCTGGTA TCTGCGACGC
1101  CGTCCGAGCA TCCGGTGTCG ACGGAGTGAT TGTCGGAAAC ACAACAAACC
1151  GTCGCCCCGA CCCTATACCC CAAGGTTACA CTCTTCCGGC CAAGGAGCAG
1201  GCAACGTTGA AAGAAACCGG CGGGTATTCA GGTCCACAGC TGTTCGATCG
1251  CACAGTGGCC CTTGTGGCTC GGTACCGCTC CATGCTGGAT GCGGAGTCGG
1301  AAACGGCCGG ATCCGCCAAG GATTCAGCAG CGACCATAGC GCAAACAGAG
1351  CCAGGCTCGG AAAACGTTCC TCCTGTGGAA GCGCCAAGCG GACTGCCGCG
1401  CAAAGTTATC TTCGCTTCGG GTGGTATCAC CAACGGGAAG CAGGCTCACG
1451  CTGTTTTAGA CACAGGGGCA TCTGTTGCCA TGATGTACAC CGGTGTGGTC
1501  TATGGTGGCG TCGGCACTGT CACTCGAGTG AAGCAAGAAC TTCGAACGGC
1551  GAAAAAGGAG.
```

12. Plasmid pGAR150.

13. A DNA compound comprising an isolated DNA sequence which imparts resistance to 8-chloro-4-(2-chloro-4-fluorophenoxy)quinoline.

14. The DNA compound of Claim 13 which is pGAR150.

15. The isolated DNA sequence of Claim 13 which is the ~7300 base pair Sall fragment of plasmid pGAR150.

16. The isolated DNA sequence of Claim 13 which is the ~5300 base pair HindIII fragment of plasmid pGAR150.

17. An isolated DNA sequence of Claim 13 which comprises a thymidyl residue at position 344 of the dihydroorotate dehydrogenase intronless-coding sequence.

18. An isolated DNA sequence of Claim 13 which comprises an adenyl residue at position 599 of the dihydroorotate dehydrogenase intronless-coding sequence.

19. A method for constructing a recombinant host cell resistant to 8-chloro-4-(2-chloro-4-fluorophenoxy)quinoline, said method comprising transforming a host cell with a recombinant DNA vector that comprises an isolated DNA sequence which imparts resistance to 8-chloro-4-(2-chloro-4-fluorophenoxy)quinoline.

20. The method of Claim 19 wherein the isolated DNA sequence is the ~7300 base pair SalI fragment of plasmid pGAR150.

21. The method of Claim 19 wherein the isolated DNA sequence is the ~5300 base pair HindIII fragment of plasmid pGAR150.

22. The method of Claim 19 wherein the host cell is a fungus.

23. The method of Claim 22 wherein the host cell is selected from the group consisting of Aspergillus, Penicillium, Cephalosporium and Neurospora.

24. The method of Claim 23 wherein the host cell is Aspergillus nidulans, Penicillium chrysogenum, Cephalosporium acremonium, or Neurospora crassa.

25. The method of Claim 19 wherein the isolated DNA sequence comprises a thymidyl residue at position 344 of the dihydroorotate dehydrogenase gene.

26. The method of Claim 19 wherein the isolated DNA sequence comprises an adenyl residue at position 599 of the dihydroorotate dehydrogenase gene.

# Figure 1

# Figure 2

# Figure 3